(19) Europäisches Patentamt
European Patent Office
Office européen des brevets

(11) Publication number: **0 686 388 A1**

(12) **EUROPEAN PATENT APPLICATION**

(21) Application number: **95107215.6**

(51) Int. Cl.[6]: **A61K 7/06**

(22) Date of filing: **12.05.95**

(30) Priority: **12.05.94 JP 98425/94**

(43) Date of publication of application:
**13.12.95 Bulletin 95/50**

(84) Designated Contracting States:
**DE GB NL**

(71) Applicant: **KAO CORPORATION**
**14-10, Nihonbashi,**
**Kayabacho 1-chome**
**Chuo-ku,**
**Tokyo (JP)**

(72) Inventor: **Ohshima, Kumi, c/o Kao Corporation**
**Research Laboratories, 1-3,**
**Bunka 2-chome**
**Sumida-ku,**
**Tokyo (JP)**

(74) Representative: **Hansen, Bernd, Dr.**
**Dipl.-Chem. et al**
**Hoffmann, Eitle & Partner,**
**Patentanwälte,**
**Arabellastrasse 4**
**D-81925 München (DE)**

(54) **Aerosol hairdressing composition**

(57) An aerosol hairdressing composition is disclosed, comprising:
(A) a cationized cellulose represented by the following formula (1):

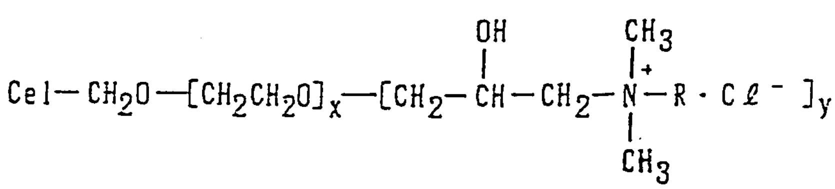

(1)

wherein R represents a $C_{6-22}$ straight-chain or branched alkyl or alkenyl group; Cel-$CH_2O$- represents a hydroxyethyl cellulose residue; x represents a number of from 0 to not more than the total number of ($CH_2OH$)'s contained in the hydroxyethyl cellulose; and y represents a number of from 1 to not more than the total number of ($CH_2OH$)'s contained in the hydroxyethyl cellulose;
(B) 0.1 to 80 % by weight of an oily component;
(C) 0 to 5 % by weight of a surfactant; and
(D) a propellent, wherein a weight ratio of the total amount of said components (A), (B) and (C) to the amount of said component (D) ranges from 99/1 to 70/30. The aerosol hairdressing composition of the present invention is excellent in foam properties such as foam expansion, fineness and retention and can exert a good effect of providing a soft, smooth and nonsticky touch.

EP 0 686 388 A1

## FIELD OF THE INVENTION

The present invention relates to an aerosol hairdressing composition. More particularly, the present invention relates to an aerosol hairdressing composition which is excellent in foam quality and can provide hairs with a soft, smooth and nonsticky touch.

## BACKGROUND OF THE INVENTION

In general, a hairdressing composition comprises various oily components for the purpose of providing hairs with a soft, smooth and moist touch. However, in the case of hairdressing compositions of types including hair oil, hair cream and stick pomade encounter problems in that the oily component shows a poor compatibility with hairs and a poor spreadability onto hairs and thus it gives a sticky touch to hair. In this point of view, an aerosol foam type hair dressing composition that provides a smooth touch of foam when applied to hair have come to be used extensively.

However, most oily components tend to inhibit foaming. Therefore, an aerosol foam type hairdressing composition needs to comprise a thickening agent or emulsifying agent in a larger amount as compared to the amount of the oily components to form good foams and hence secure the smooth use. However, if a thickening agent or emulsifying agent is used in such a large amount, an effect inherent to the oily component of providing a soft or smooth touch is impaired. Further, the amount of the oily component to be blended can be relatively and absolutely restricted. Thus, the oily component cannot be incorporated in an amount large enough to provide hairs with a desirable touch. Accordingly, it has been difficult to obtain an aerosol hairdressing composition without impairing properties inherent to the oily components.

## SUMMARY OF THE INVENTION

It is therefore an object of the present invention to provide an aerosol hairdressing composition which exhibits an excellent foam quality and can provide hairs with a soft, smooth and nonsticky touch.

Under such circumstances, the inventors made extensive studies of the foregoing problems. As a result, it was found that the use of a specific cationized cellulose can minimize the amount of a thickening agent or emulsifying agent to be used and enables the blending of an oily component in an amount large enough to provide hairs with a desirable touch such as softness and smoothness without impairing the foamability of the hairdressing composition. Thus, the present invention has been completed.

Namely, the present invention provides an aerosol hairdressing composition comprising:

(A) 0.01 to 5 % by weight of a cationized cellulose represented by the following formula (1):

$$Cel-CH_2O-[CH_2CH_2O]_x-[CH_2-CH(OH)-CH_2-N^{+}(CH_3)_2-R\cdot Cl^{-}]_y \quad (1)$$

wherein R represents a $C_{6-22}$ straight-chain or branched alkyl or alkenyl group; Cel-$CH_2O$- represents a hydroxyethyl cellulose residue; x represents a number of from 0 to not more than the total number of ($CH_2OH$)'s contained in the hydroxyethyl cellulose; and y represents a number of from 1 to not more than the total number of ($CH_2OH$)'s contained in the hydroxyethyl cellulose;

(B) 0.1 to 80 % by weight of an oily component;

(C) 0 to 5 % by weight of a surfactant; and

(D) a propellent,

wherein a weight ratio of the total amount of said components (A), (B) and (C) to the amount of said component (D) ranges from 99/1 to 70/30.

## DETAILED DESCRIPTION OF THE INVENTION

The cationized cellulose as component (A) to be used in the present invention is represented by the foregoing formula (1). In formula (1), R is preferably a group having 8 to 16 carbon atoms. Examples of the $C_{6-22}$ straight-chain alkyl group represented by R include hexyl group, heptyl group, octyl group, nonyl

group, decyl group, undecyl group, dodecyl group, tridecyl group, tetradecyl group, pentadecyl group, heptadecyl group, hexadecyl group, nonadecyl group, and icosyl group. Examples of the $C_{6-22}$ branched alkyl group represented by R include 2-ethylhexyl group, and 2-heptylundecyl group. Examples of the $C_{6-22}$ alkenyl group include oleyl group, palmitoleyl group, and undecenyl group. Among them, $C_{6-22}$ alkyl groups are preferred and dodecyl group is more preferred.

In formula (1), x and y are preferably from 0 to 20,000 and from 1 to 20,000, and more preferably from 50 to 20,000 and from 50 to 20,000, respectively.

As the cationized cellulose to be used as component (A), there may be used those described in Cosmetics & Toiletries 108 (May), p. 106 (1993), JP-A-61-181801 (The term "JP-A" as used herein means an "unexamined published Japanese patent application") and U.S. Patent 4,663,159, hereby incorporated by reference. For example, commercially available products such as QUATRISOFT POLYMER LM-200 (available from Union Carbide Inc.) may be used.

The preparation of the cationized cellulose as component (A) represented by formula (1) can be prepared by the reaction of hydroxyethyl cellulose with a monoalkyl ($C_{6-22}$) dimethylammonium epoxide.

The amount of the cationized cellulose to be incorporated as component (A) is preferably from 0.01 to 5 % by weight, more preferably from 0.1 to 5 % by weight, and still more preferably from 1 to 3 % by weight, based on the total weight of the composition.

As the oily component to be incorporated as component (B), there may be used one which is commonly incorporated in hair cosmetics. For example, hydrocarbons, glycerides, waxes, higher alcohols, esters, higher fatty acids, silicone derivatives, and the like may be used. These oily components may be used singly or in combination.

Examples of the hydrocarbon employable in the present invention include squalene, squarane, liquid paraffin, liquid isoparaffin, and cycloparaffin. Examples of the glyceride employable in the present invention include castor oil, cocoa oil, mink oil, avocado oil, and olive oil. Examples of the wax employable in the present invention include beeswax, whale wax, lanolin, and carnauba wax. Examples of the higher alcohol employable in the present invention include cetyl alcohol, oleyl alcohol, stearyl alcohol, isostearyl alcohol, 2-octyl dodecanol, propylene glycol, and glycerin. Examples of the ester employable in the present invention include octyldodecyl myristate, hexyl laurate, emulsified cetyl, propyleneglycol monostearate, oleyl oleate, hexadecyl 2-ethylhexanoate, isononyl isononanoate, tridecyl isononanoate, and cholesteryl isostearate. Examples of the higher fatty acid include capric acid, lauric acid, myristic acid, palmitic acid, stearic acid, behenic acid, oleic acid, coconut oil fatty acid, isostearic acid, and isopalmitic acid. Other examples include isostearyl glyceryl ether, and polyoxypropylene butyl ether.

As the silicone derivatives, there may be used one or more of compounds represented by the following formulae (a) to (g):

(a) Polyether-modified silicone represented by the following formulae (2) to (5):

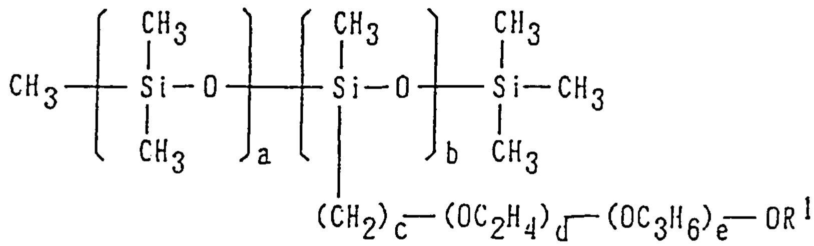

(2)

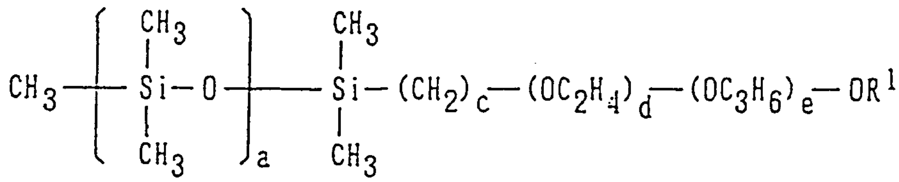

(3)

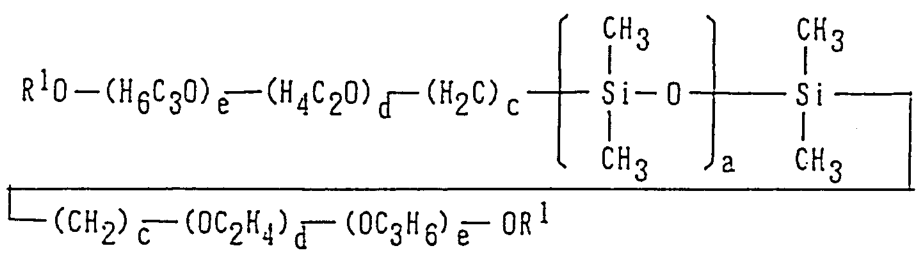

(4)

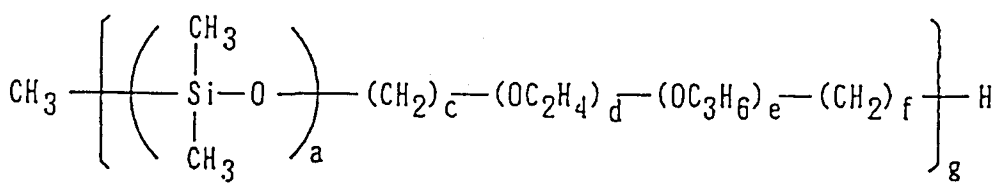

(5)

wherein $R^1$ represents hydrogen atom or a $C_{1-10}$ hydrocarbon; a represents a number of 1 or more; b represents a number of 1 or more; c represents a number of from 1 to 6; d represents a number of from 1 to 300; e represents a number of from 0 to 300; f represents a number of from 0 to 6; and g represents a number of from 2 to 500.

The hydrocarbon group represented by $R^1$ is preferably a $C_{1-10}$ straight-chain or branched alkyl group such as methyl, ethyl, propyl, butyl, hexyl, octyl and decyl, particularly methyl or butyl, a is preferably a number of from 1 to 100, b is preferably a number from 1 to 50, c is preferably a number of from 1 to 6, d is preferably a number of from 2 to 50, e is preferably a number of from 0 to 50, f is preferably a number of from 0 to 6, and g is preferably a number of from 2 to 500.

Preferred among these polyether-modified silicones are side-chain polyether-modified silicone represented by formula (2) and double-end polyether-modified silicone represented by formula (4), particularly one represented by formula (2).

(b) Dimethyl polysiloxane, methylphenyl polysiloxane and diphenyl polysiloxane represented by the following formula (6):

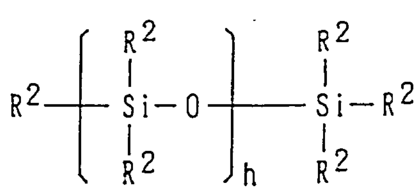

(6)

wherein $R^2$'s may be the same or different and each represents methyl group, phenyl group or -OSi-

$(CH_3)_3$; and h represents a number of 2 or more.

In the foregoing formula (6), $R^2$ is preferably methyl group or phenyl group, and h is preferably a number of from 3 to 20,000.

Particularly preferred among these polysiloxanes are dimethyl polysiloxane, and methylphenyl polysiloxane.

(c) Long chain alkyl-modified silicone represented by the following formula (7):

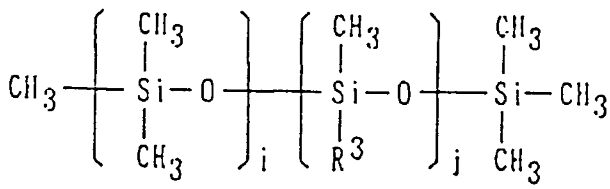 (7)

wherein $R^3$ represents a $C_{10-50}$ alkyl group; i represents a number of from 0 to 1,000; and j represents a number of from 0 to 1,000.

In the foregoing formula (7), $R^3$ is preferably a $C_{15-40}$ straight-chain or branched alkyl group, i is preferably a number of from 10 to 500, and j is preferably a number of from 10 to 500.

(d) Alkoxy-modified silicone represented by the following formula (8):

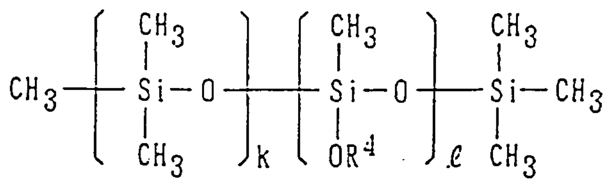 (8)

wherein $R^4$ represents a $C_{10-50}$ hydrocarbon group; k represents a number of from 3 to 100; and 1 represents a number of from 1 to 50.

In the foregoing formula (8), the hydrocarbon group represented by $R^4$ is preferably a $C_{12-20}$ straight-chain or branched alkyl group, k is preferably a number of from 5 to 50, and 1 is preferably a number of from 2 to 20.

(e) Amino-modified silicone represented by the following formula (9):

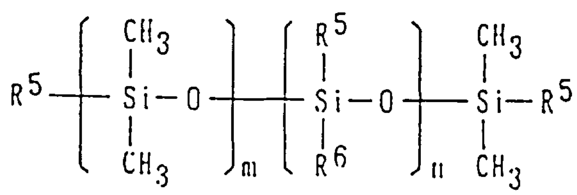 (9)

wherein $R^5$'s may be the same or different and each represents hydrogen atom, hydroxyl group, methyl group or methoxy group; $R^6$ represents $-(CH_2)_o-(OCH_2H_4)_p-(OC_3H_6)_q-(NHC_2H_4)N(R^7)_6$ or $-(CH_2)_o-(OCH_2H_4)_p-(OC_3H_6)_q-(NHC_2H_4)N^+(R^7)_3 \cdot Z^-$(in which $R^7$'s may be the same or different and each represents hydrogen atom or a $C_{1-6}$ hydrocarbon group, $Z^-$ represents a halogen ion or an organic anion, o represents a number of from 1 to 6, and p and q each represents a number of from 0 to 6); m represents a number of from 3 to 300; and n represents a number of from 1 to 50.

In the foregoing formula (9), m is preferably a number of from 5 to 150, and n is preferably a number of from 1 to 20.

(f) Bunte salt-modified silicone represented by the following formula (10) or (11):

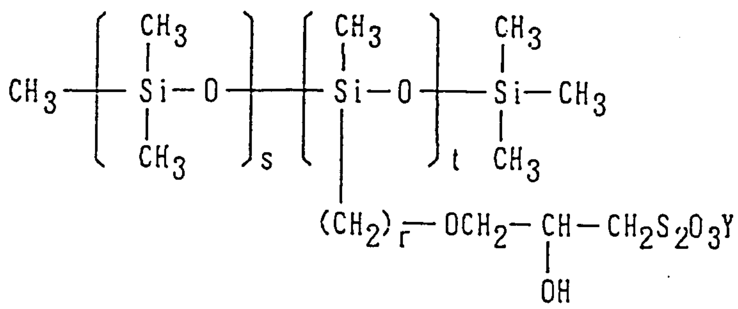

(10)

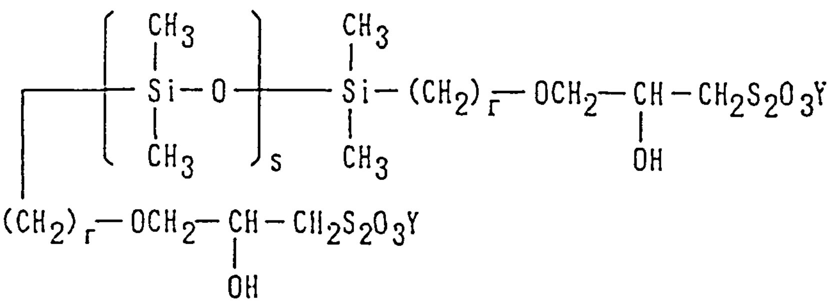

(11)

wherein Y represents an alkaline metal, an alkaline earth metal, an amine or a quaternary ammonium salt; r represents a number of from 1 to 20; and s and t each represents a number of from 1 to 20, with the proviso that s and t satisfy the relationship $s/t > 1$.

In the foregoing formulae (10) and (11), Y is preferably sodium, potassium, calcium, barium, ammonia or an ammonium salt, r is preferably a number of from 3 to 12, and s and t each is preferably a number of from 2 to 18.

(g) Silicone resin represented by the following formula (12):

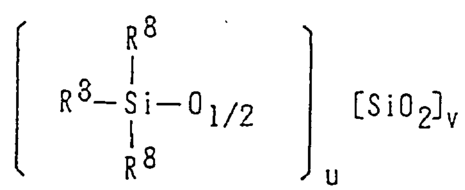

(12)

wherein $R^8$'s may be the same or different and each represents methyl group or phenyl group; and u and v satisfy the relationship $u/v = 0.1$ to 10.

In the formula (12), u and v each is preferably a number of from 0.5 to 5.

Particularly preferred among these silicone derivatives are polyether-modified silicone, dimethyl polysiloxane, methylphenyl polysiloxane, and amino-modified silicone.

The amount of the oily component to be incorporated as component (B) is preferably from 0.1 to 80 % by weight based on the total weight of the composition. In particular, the oily component may preferably be incorporated in an amount of from 0.5 to 50 % by weight, more preferably from 1 to 30 % by weight, based on the total weight of the composition, to provide a better touch.

As to the surfactant (C), there may be used one which is commonly used in cosmetics. For example, anionic, cationic, amphoteric and nonionic surfactants may be used in the present invention. The amounts of the surfactant (C) in the hairdressing composition of the present invention is preferably from 0 to 5 % by weight, based on the total weight of the composition.

As the propellent (D), there may be used one which is commonly used in aerosol cosmetics. For example, lower saturated hydrocarbons such as propane, butane and mixture thereof (including liquefied petroleum gas), ethers such as dimethylether, nitrogen gas, carbon dioxide gas, nitrous oxide gas, and the like may be used. These propellents may be used singly or in combination.

The propellent (D) is preferably incorporated in the hairdressing composition of the present invention in an amount to give a weight ratio of the total amount of the cationized cellulose (A), the oily component (B)

and the surfactant (C) to the propellent (D) ranging from 99/1 to 70/30. It is preferred that the amount of the propellent (D) in the hairdressing composition of the present invention ranges from 0.01 to 50 % by weight, more preferably from 5 to 20 % by weight, based on the total weight of the composition.

Besides the foregoing components (A) to (D), the aerosol hairdressing composition of the present invention may comprise components which are commonly incorporated in aerosol type hairdressing cosmetics in such an amount that the effects of the present invention are not impaired. Examples of these components include humectants, pharmaceuticals, ultraviolet absorbents, preservatives, antioxidants, pigments, dispersants, metallic ion-blocking agents, lower alcohols, crude drug extracts, mucopolysaccharides, amino acids, proteins, dyes, and perfumes. These components are preferably incorporated in the composition in the form of solution or dispersion in water.

The aerosol hairdressing composition of the present invention may preferably be used in the form of cosmetic that can be directly applied to hairs without being removed by rinsing, such as setting lotion, blow styling lotion, styling agent, hair treatment agent, hair mist and hair cream.

The aerosol hairdressing composition of the present invention may be prepared in conventional manner. When an oily component having a high viscosity is employed, it may be dissolved or mixed with another oily component having a low viscosity in advance and then blended with the other components.

The aerosol hairdressing composition of the present invention comprises a cationized cellulose represented by formula (1) and thus requires none or minimized amount of a thickening agent or emulsifying agent which is commonly necessary for aerosol hairdressing composition. Thus, the aerosol hairdressing composition of the present invention can thoroughly exert an effect of providing an excellent foam quality and a soft and smooth touch inherent to the oily component. Even if the aerosol hairdressing composition comprises an oily component in an amount large enough to provide hairs with a desirable touch, it can retain excellent foam properties and doesn't give a sticky touch.

The present invention will be further described in the following examples, but the present invention should not be construed as being limited thereto.

## EXAMPLE 1

Among the components set forth in Table 1, the components other than propellent were mixed and dissolved to prepare stock solutions. To the stock solutions was each then added the respective propellent to prepare desired aerosol hairdressing compositions. These aerosol hairdressing compositions were then evaluated for foam properties (foam expansion, fineness, retention) and touch (softness of hair, smoothness of hair, nonstickiness of hair). The results are set forth in Table 1.

### Evaluation method

(Foam expansion)

A foam was sprayed on to a plane from a container. The expansion of the foam which had been immediately developed was evaluated by the ratio of the diameter of the foam thus expanded to the height of the foam thus expanded in accordance with the following criteria:

A: (Height ÷ expanded diameter) ≧ 0.75
B: 0.60 ≦ (Height ÷ expanded diameter) < 0.75
C: 0.50 ≦ (Height ÷ expanded diameter) < 0.60
D: (Height ÷ expanded diameter) < 0.50

(Foam fineness)

A foam which had been sprayed on to a plane from the vessel was immediately evaluated for fineness in accordance with the following criteria.

A: Fine, white and creamy appearance
B: Fine, but many holes observed in foam
C: Transparent foam membranes observed
D: Many transparent foam membranes observed/foams floated on liquid

(Foam retention)

A foam about the size of a table tennis ball was sprayed from the vessel. The time required until the foam disappears and liquefies at room temperature was evaluated in accordance with the following criteria. In the evaluation on a palm, a foam which had been sprayed was repeatedly cut by fingers. For the static evaluation, a foam which had been sprayed on to a plane was allowed to stand.

- A: The foam remains over 10 seconds in the evaluation on a palm and over 10 minutes in the static evaluation;
- B: The foam remains over 10 seconds in the evaluation on a palm but disappears and liquefies within 10 minutes or less in the static evaluation;
- C: The foam disappears and liquefies within 10 seconds or less in the evaluation on a palm;
- D: The foam liquefies immediately after being sprayed

(Touch)

Middle-length human hair wigs were each washed with a standard shampoo, and then dried with a towel. The hairs, which had been still dampened, were then uniformly coated with 5 g of the hairdressing composition. These wigs were each then dried and set with a dryer and a brush by a panel of female experts. As compared with wigs finished uncoated, these wigs thus finished were each then organoleptically evaluated for various properties according to the following criteria:

Softness:

- A: Very softer than uncoated;
- B: Softer than uncoated;
- C: Soft as much as uncoated;
- D: Less soft than uncoated

Smoothness:

- A: Very smoother than uncoated;
- B: Smoother than uncoated;
- C: Smooth as much as uncoated;
- D: Less smooth than uncoated

Nonstickiness:

- A: Less sticky than uncoated;
- B: Sticky as much as uncoated;
- C: Stickier than uncoated

TABLE 1

| Ingredient (% by weight) | Product of the Invention 1 | Product of the Invention 2 | Comparative Product 1 | Comparative Product 2 | Comparative Product 3 | Comparative Product 4 |
|---|---|---|---|---|---|---|
| (Stock solution) | | | | | | |
| Cationized cellulose (LM-200, available from Union Carbide Inc.) | 1.0 | 1.0 | - | - | - | - |
| 2-Octyl dodecanol | 30.0 | - | - | - | - | - |
| Liquid isoparaffin | - | 30.0 | 30.0 | 30.0 | 30.0 | 30.0 |
| Stearyltrimethylammonium chloride | - | - | 1.0 | - | - | 1.0 |
| Cationized cellulose derivative (Polymer JR-400, available from Union Carbide Inc.) | - | - | - | 0.5 | - | - |
| Hydroxyethyl cellulose | - | - | - | - | 0.5 | 0.5 |
| 95 % Ethanol | 10.0 | 10.0 | 10.0 | 10.0 | 10.0 | 10.0 |
| Purified water | balance | balance | balance | balance | balance | balance |
| (Propellent) Liquefied petroleum gas/dimethyl ether | Stock solution/propellent = 80/20 | | Stock solution/propellent = 90/10 | | | |
| Foam properties: Expansion | A | A | D | C | D | C |
| Fineness | A | A | B | D | D | B |
| Retention | A | A | C | D | D | C |
| Touch: Softness | A | A | B | C | D | D |
| Smoothness | A | A | B | B | C | C |
| Nonstickiness | A | A | C | C | C | C |

As can be seen in Table 1, the aerosol hairdressing compositions of the present invention exhibit excellent foam properties although they comprised no thickening agent or emulsifying agent incorporated therein or comprised such an agent, if any, in a minimized amount. Further, since the composition of the present invention are free of thickening agent or emulsifying agent, the effect inherent to the oily component of providing a touch such as softness can be thoroughly exerted.

EXAMPLE 2

Aerosol hairdressing compositions having the formulations set forth in Tables 2 and 3 were prepared in the same manner as in Example 1.

TABLE 2

| Ingredient (% by weight) | Product of the Invention | | | | | | |
|---|---|---|---|---|---|---|---|
| | 3 | 4 | 5 | 6 | 7 | 8 | 9 |
| (Stock solution) | | | | | | | |
| Cationized cellulose (LM-200, available from Union Carbide Inc.) | 0.2 | 0.5 | 0.7 | 1.0 | 1.0 | 1.5 | 1.5 |
| Cetostearyl alcohol | - | - | - | - | 0.2 | - | 0.5 |
| Octyldodecyl myristate | - | - | - | - | - | - | 0.3 |
| Cholesteryl hydroxystearate | - | 2.0 | - | - | - | - | - |
| Liquid isoparaffin | 5.0 | 30.0 | 15.0 | - | 20.0 | 20.0 | - |
| Liquid paraffin | 1.0 | - | - | - | - | - | - |
| Isononyl isononaoate | - | - | - | 10.0 | - | - | - |
| Stearic acid | - | - | - | - | - | 0.1 | - |
| Polyoxypropylene butyl ether | - | - | - | - | - | - | - |
| Stearyltrimethylammonium chloride | - | 0.05 | 0.1 | - | - | - | 0.1 |
| Polyoxyethylene (20) stearyl ether | 0.2 | - | - | - | - | - | - |
| Triethanolamine | - | - | - | - | - | Slight amount | - |
| 95 % Ethanol | 10.0 | 10.0 | 15.0 | 20.0 | 10.0 | 20.0 | 20.0 |
| Purified water | balance | balance | balance | balance | balance | balance | balance |
| (Propellent) Liquefied petroleum gas | 100.0 | 60.0 | 90.0 | 90.0 | 80.0 | 70.0 | 100.0 |
| Dimethyl ether | - | 40.0 | 10.0 | 10.0 | 20.0 | 30.0 | - |
| Stock solution/propellent | 90/10 | 85/15 | 90/10 | 90/10 | 85/15 | 85/15 | 90/10 |

TABLE 3

| Ingredient (% by weight) | Product of the Invention 10 | 11 | 12 | 13 | 14 | 15 | 16 |
|---|---|---|---|---|---|---|---|
| (Stock solution) | | | | | | | |
| Cationized cellulose (LM-200, available from Union Carbide Inc.) | 1.5 | 2.0 | 2.0 | 2.5 | 2.5 | 3.0 | 3.0 |
| Cetostearyl alcohol | - | - | - | - | - | - | - |
| 2-Ocyldodecanol | 0.5 | - | - | 0.1 | - | 30.0 | - |
| Octyldodecyl myristate | 0.3 | 1.0 | 0.3 | - | - | - | - |
| Cholesteryl hydroxystearate | - | - | - | - | - | - | 0.5 |
| Liquid isoparaffin | - | 15.0 | - | 50.0 | - | - | - |
| Liquid paraffin | - | - | - | - | - | - | - |
| Isononyl isononaoate | - | - | - | - | - | - | - |
| Stearic acid | - | - | - | - | - | - | - |
| Polyoxypropylene butyl ether | - | - | 10.0 | - | 0.5 | - | - |
| Stearyltrimethylammonium chloride | - | - | 0.3 | 0.1 | - | - | - |
| Polyoxyethylene (20) stearyl ether | 0.2 | 0.2 | - | - | - | - | - |
| Triethanolamine | - | - | - | - | - | - | - |
| 95 % Ethanol | 20.0 | 10.0 | 20.0 | 5.0 | 10.0 | 20.0 | 30.0 |
| Purified water | balance | balance | balance | balance | balance | balance | balance |
| (Propellent) Liquefied petroleum gas | 100.0 | 70.0 | 90.0 | 60.0 | 100.0 | 70.0 | 80.0 |
| Dimethyl ether | - | 30.0 | 10.0 | 40.0 | - | 30.0 | 20.0 |
| Stock solution/propellent | 90/10 | 90/10 | 90/10 | 80/20 | 90/10 | 93/7 | 90/10 |

These aerosol hairdressing compositions thus obtained were evaluated in the same manner as in Example 1. As a result, it was found that these aerosol hairdressing compositions exhibit excellent foam properties and touch.

EXAMPLE 3

Among the components set forth in Tables 4 to 6, the components other than propellent were mixed and dissolved to prepare stock solutions. To these stock solutions were each then added the respective propellent. These mixtures were each charged into a pressure container to prepare aerosol hairdressing compositions. These aerosol hairdressing compositions were evaluated for foam properties (foam expansion, fineness, retention) and touch (softness of hair, smoothness of hair, nonstickiness of hair) in the same manner as in Example 1. The results are set forth in Table 3.

TABLE 4

| Ingredient (% by weight) | | Product of the Invention | | | | Comparative product | | | |
|---|---|---|---|---|---|---|---|---|---|
| | | 17 | 18 | 19 | 20 | 5 | 6 | 7 | 8 |
| (Stock solution) | | | | | | | | | |
| Polyether-modified silicone*1 | | 0.5 | - | - | - | 0.5 | - | - | - |
| Dimethyl polysiloxane*2 | | - | 5.0 | - | 2.0 | - | - | 5.0 | 2.0 |
| Methylphenyl silicone*3 | | 1.0 | - | - | - | 1.0 | - | - | - |
| Liquid isoparaffin | | - | - | - | 5.0 | - | - | - | 5.0 |
| Cationized cellulose*4 | | 0.5 | 1.0 | 0.2 | 1.5 | - | - | - | - |
| Stearyltrimethylammonium chloride | | - | - | - | - | 1.5 | - | - | 1.0 |
| Polyoxyethylene alkyl ether (9EO)*5 | | - | - | 0.5 | - | - | - | 0.5 | - |
| Cationized cellulose derivative*6 | | - | - | - | - | - | 0.5 | - | - |
| Hydroxyethyl cellulose*7 | | - | - | - | - | - | - | 0.2 | 0.1 |
| 95 % Ethanol | | 10.0 | 10.0 | 10.0 | - | 10.0 | 10.0 | 10.0 | - |
| Purified water | | balance | balance | balance | balance | balance | balance | balance | balance |
| (Propellent) | Liquefied petroleum gas | Stock solution/propellent = 90/10 | | | | | | | |
| Foam properties: | Expansion | A | A | A | A | B | C | D | C |
| | Fineness | A | A | A | A | C | C | D | B |
| | Retention | A | A | A | A | D | D | D | D |
| Touch: | Softness | A | A | A | A | A | B | C | C |
| | Smoothness | A | A | A | A | A | B | B | B |
| | Nonstickiness | A | A | A | A | C | C | C | C |

TABLE 5

| Ingredient (% by weight) | | Product of the Invention | | | | Comparative product | | | |
|---|---|---|---|---|---|---|---|---|---|
| | | 21 | 22 | 23 | 24 | 9 | 10 | 11 | 12 |
| (Stock solution) | | | | | | | | | |
| Dimethyl polysiloxane*8 | | - | - | - | 0.5 | - | - | - | 0.5 |
| Long chain alkyl-modified silicone*9 | | 1.0 | - | - | - | 1.0 | - | - | - |
| Stearoxy-modified silicone*10 | | - | 1.0 | - | - | - | 1.0 | - | - |
| Amino-modified silicone*11 | | - | - | 1.0 | - | - | - | 1.0 | - |
| Silicone resin*12 | | - | - | - | 5.0 | - | - | - | 0.5 |
| Liquid paraffin | | - | - | - | 7.0 | - | - | - | 7.0 |
| Behenyl alcohol | | 0.5 | 5.0 | - | - | 0.5 | 5.0 | - | - |
| Cationized cellulose*4 | | 0.5 | 1.0 | 0.2 | 1.5 | - | - | - | - |
| Stearyltrimethylammonium chloride | | - | - | - | 0.5 | - | 1.0 | - | - |
| Polyoxyethylene alkyl ether (9EO)*5 | | - | - | - | - | 0.5 | - | - | 1.0 |
| Cationized cellulose derivative*6 | | - | - | - | - | - | - | - | 0.1 |
| Hydroxyethyl cellulose | | - | - | - | - | - | 0.2 | 0.2 | - |
| 95 % Ethanol | | 5.0 | 10.0 | 10.0 | 10.0 | 10.0 | 10.0 | 10.0 | 10.0 |
| Purified water | | balance | balance | balance | balance | balance | balance | balance | balance |
| (Propellent) Liquefied petroleum gas | | Stock solution/propellent = 90/10 | | | | | | | |
| Foam properties: | Expansion | A | A | A | A | C | C | C | C |
| | Fineness | A | A | A | A | C | D | C | C |
| | Retention | A | A | A | A | C | D | D | C |
| Touch: | Softness | A | A | A | A | A | C | B | C |
| | Smoothness | A | A | A | A | A | B | A | A |
| | Nonstickiness | A | A | A | A | B | A | B | A |

TABLE 6

| Ingredient (% by weight) | | Product of the Invention | | | Comparative product | | | |
|---|---|---|---|---|---|---|---|---|
| | | 25 | 26 | 27 | 13 | 14 | 15 | 16 |
| (Stock solution) | | | | | | | | |
| Dimethyl polysiloxane*13 | | 2.0 | 2.0 | 2.0 | 2.0 | 2.0 | 2.0 | 2.0 |
| Light liquid paraffin | | 15.0 | 15.0 | 15.0 | 15.0 | 15.0 | 15.0 | 15.0 |
| Cationized cellulose*4 | | 1.5 | 1.0 | 0.2 | - | - | - | - |
| Stearyltrimethylammonium chloride | | - | - | - | - | 1.0 | - | - |
| Polyoxyethylene alkyl ether (9EO)*5 | | - | - | 1.0 | 1.0 | - | - | - |
| 2-Alkyl-N-carboxymethyl-N-hydroxyethyl imidazoliniumbetain*14 | | - | - | - | - | - | 1.0 | 1.0 |
| Stearic acid | | - | - | - | - | - | - | 0.5 |
| 95 % Ethanol | | 5.0 | 5.0 | 5.0 | 5.0 | 5.0 | 5.0 | 5.0 |
| Purified water | | balance | balance | balance | balance | balance | balance | balance |
| (Propellent) Liquefied petroleum gas | | Stock solution/propellent = 90/10 | | | | | | |
| Foam properties: | Expansion | A | A | A | B | B | B | B |
| | Fineness | A | A | A | D | D | B | B |
| | Retention | A | A | A | D | D | D | B |
| Touch: | Softness | A | A | A | - | - | C | D |
| | Smoothness | A | A | A | - | - | B | B |
| | Nonstickiness | A | A | A | - | - | B | C |

Notes:

*1: KF6005, available from Shin-Etsu Chemical Co., Ltd.

*2: 5cs

*3: KF-54, available from Shin-Etsu Chemical Co., Ltd.

*4: LM-200, available from Union Carbide Inc.

*5: Softanol 90, available from Nippon Shokubai Co., Ltd.

*6: Polymer JR400, available from Union Carbide Inc.

*7: HEC Daicel SE850, available from Daicel Chemical Industries, Ltd.

*8: 30Fcs

*9: TSF4420, available from Toshiba Silicone Co., Ltd.

*10: KF7002, available from Shin-Etsu Chemical Co., Ltd.

*11: Silicone SM8702C, available from Toray Industries, Inc.

*12: KF7312K, available from Shin-Etsu Chemical Co., Ltd.

*13: 50cs/1,000,000cs mixture

*14: Obazoline 552, available from Toho Chemical Co., Ltd.

As can be seen in Tables 4 to 6, the aerosol hairdressing compositions of the present invention exhibit excellent foam properties and can thoroughly exert an effect inherent to the oily component such as silicone derivative of providing a soft touch or the like.

While the invention has been described in detail and with reference to specific embodiments thereof, it will be apparent to one skilled in the art that various changes and modifications can be made therein without departing from the spirit and scope thereof.

## Claims

**1.** An aerosol hairdressing composition comprising:

(A) 0.01 to 5 % by weight of a cationized cellulose represented by the following formula (1):

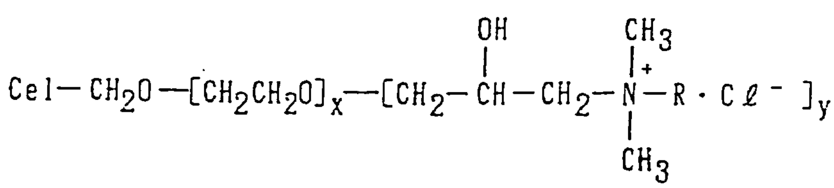

(1)

wherein R represents a $C_{6-22}$ straight-chain or branched alkyl or alkenyl group; Cel-$CH_2O$- represents a hydroxyethyl cellulose residue; x represents a number of from 0 to not more than the total number of $(CH_2OH)$'s contained in hydroxyethyl cellulose; and y represents a number of from 1 to not more than the total number of $(CH_2OH)$'s contained in hydroxyethyl cellulose;

(B) 0.1 to 80 % by weight of an oily component;

(C) 0 to 5 % by weight of a surfactant; and

(D) a propellent,
wherein a weight ratio of the total amount of said components (A), (B) and (C) to the amount of said component (D) ranges from 99/1 to 70/30.

**2.** The aerosol hairdressing composition of claim 1, wherein said R is a dodecyl group.

**3.** The aerosol hairdressing composition of claim 1, wherein said oily component (B) is at least one compound selected from the group consisting of hydrocarbons, glycerides, waxes, higher alcohols, esters, higher fatty acids and silicone derivatives.

**4.** The aerosol hairdressing composition of claim 1, wherein said cationized cellulose (A) and said oily component (B) are present in amounts of from 0.1 to 5 % by weight and from 1 to 30 % by weight, respectively.

European Patent Office

# EUROPEAN SEARCH REPORT

Application Number
EP 95 10 7215

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| P,X | FR-A-2 706 296 (L'OREAL) 23 December 1994<br>* the whole document *<br>--- | 1-4 | A61K7/06 |
| X | WO-A-93 08787 (THE PROCTER & GAMBLE COMPANY)<br>* the whole document *<br>--- | 1-4 | |
| A | WO-A-88 05812 (REVLON INC.)<br>* the whole document *<br>--- | 1-4 | |
| A | EP-A-0 149 249 (UNION CARBIDE CORPORATION)<br>* the whole document *<br>--- | 1-4 | |
| A | EP-A-0 189 935 (UNION CARBIDE CORPORATION)<br>* the whole document *<br>----- | 1-4 | |

TECHNICAL FIELDS SEARCHED (Int.Cl.6)

A61K

The present search report has been drawn up for all claims

| Place of search | Date of completion of the search | Examiner |
|---|---|---|
| THE HAGUE | 19 September 1995 | Couckuyt, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document